(19) Europäisches Patentamt
European Patent Office
Office européen des brevets

(11) **EP 1 732 448 B1**

(12) **FASCICULE DE BREVET EUROPEEN**

(45) Date de publication et mention
de la délivrance du brevet:
**21.11.2007 Bulletin 2007/47**

(21) Numéro de dépôt: **05744163.6**

(22) Date de dépôt: **25.03.2005**

(51) Int Cl.:
*A61B 17/02* *(2006.01)*      *A61B 17/17* *(2006.01)*

(86) Numéro de dépôt international:
**PCT/FR2005/000726**

(87) Numéro de publication internationale:
**WO 2005/092205 (06.10.2005 Gazette 2005/40)**

(54) **ENSEMBLE D ANCILLAIRES POUR IMPLANTER UNE PROTHESE DE GENOU**

HILFSANORDNUNG ZUR IMPLANTATION VON KNIEPROTHESEN

ANCILLARY ASSEMBLY FOR IMPLANTING A KNEE PROSTHESIS

(84) Etats contractants désignés:
**AT BE BG CH CY CZ DE DK EE ES FI FR GB GR
HU IE IS IT LI LT LU MC NL PL PT RO SE SI SK TR**

(30) Priorité: **26.03.2004 FR 0403156**

(43) Date de publication de la demande:
**20.12.2006 Bulletin 2006/51**

(73) Titulaire: **Guillaume, Francis**
**71410 Sanvignes Les Mines (FR)**

(72) Inventeur: **Guillaume, Francis**
**71410 Sanvignes Les Mines (FR)**

(74) Mandataire: **Grand, Guillaume et al**
**Cabinet Lavoix**
**62, rue de Bonnel**
**68448 Lyon Cedex 03 (FR)**

(56) Documents cités:
**WO-A-00/78225          WO-A-01/85038**
**FR-A- 2 587 198          US-A- 5 649 929**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne un ensemble d'ancillaires pour implanter une prothèse de genou, notamment une prothèse dite « à plateau mobile ».

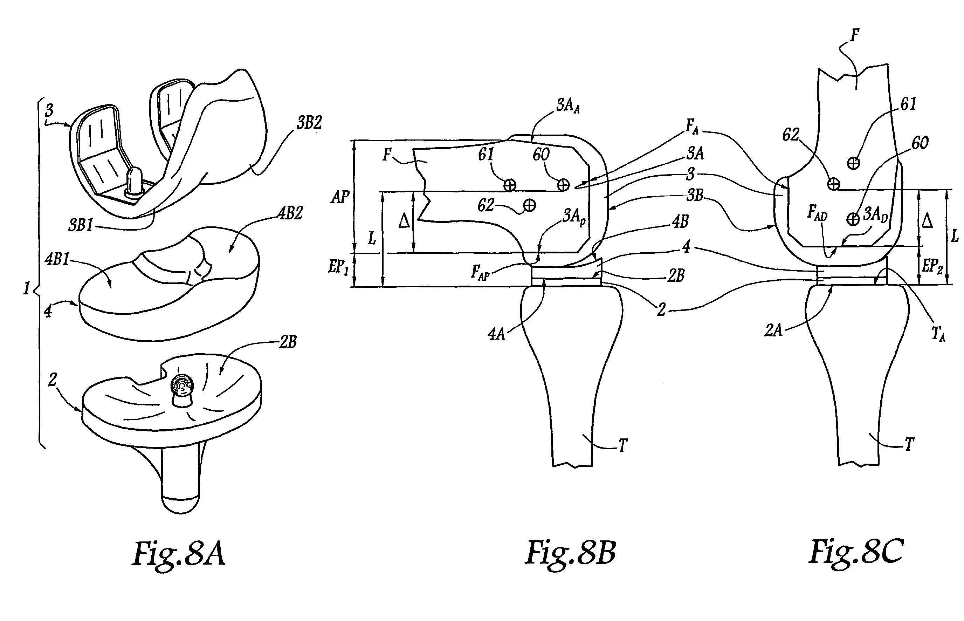

Sur la figure 8A est représenté un exemple 1 d'une telle prothèse de genou « à plateau mobile ». De manière connue, cette prothèse 1 comporte un composant tibial sous forme d'une embase métallique 2, un composant fémoral sous forme d'une coiffe métallique 3 et un insert 4 en polyéthylène constituant le plateau mobile de la prothèse. Comme représenté sur les figures 8B et 8C, l'embase tibiale 2 est prévue pour être solidarisée fermement à l'extrémité supérieure du tibia T et délimite à cet effet une face 2A d'appui sur et de solidarisation à une surface réséquée correspondante $T_A$ du tibia tandis que la coiffe fémorale 3 est solidarisée fermement à l'extrémité inférieure du fémur F en délimitant à cet effet une face multi-plans 3A d'appui sur et de solidarisation à une face réséquée correspondante $F_A$ du fémur. La face 4A du plateau 4 tournée vers l'embase 2 repose de façon mobile à la face supérieure 2B de cette embase tandis que la face opposée 4B du plateau définit deux surfaces concaves $4B_1$ et $4B_2$ destinées à recevoir de manière articulée deux surfaces convexes associées $3B_1$ et $3B_2$ définies par la face correspondante 3B de la coiffe 3 et reproduisant approximativement la géométrie de deux condyles fémoraux anatomiques interne et externe.

La prothèse 1 permet de reproduire une cinématique proche de celle du genou anatomique, garantissant au patient un bon confort en utilisation. Cependant, les libertés de mouvements internes à cette prothèse nécessitent une grande précision d'implantation des composants prothétiques et obligent à tenir compte de l'environnement ligamentaire du genou. En particulier, afin d'assurer les meilleures amplitudes articulaires possibles et la meilleure stabilité possible, le chirurgien recherche autant que possible l'égalité des valeurs de l'écartement en utilisation entre le fémur et le tibia, tant en flexion, notamment à 90°, qu'en extension. Cet écartement correspond en pratique à la place dont disposent les parties prothétiques en cours de sollicitation, c'est-à-dire les parties de la prothèse en contact articulé les unes contre les autres qui, de par leur dimensionnement, présentent une épaisseur totale, suivant la direction de sollicitation, sensiblement identique en flexion à 90° et en extension. Cet écartement est, de ce fait, couramment désigné par l'expression « encombrement prothétique ». Ces encombrements prothétiques en flexion et en extension sont respectivement notés $EP_1$ et $EP_2$ sur les figures 8B et 8C, l'encombrement prothétique $EP_1$ en flexion étant sensiblement égal à la distance entre la surface tibiale réséquée $T_A$ et le plan de coupe postérieure $F_{AP}$ de la face fémorale réséquée $F_A$, tandis que l'encombrement prothétique $EP_2$ en extension est sensiblement égal à la distance entre la même surface tibiale $T_A$ et le plan de coupe distale $F_{AD}$ de la face fémorale multi-plans $F_A$.

De plus, pour éviter tout excès de pression sur un des côtés interne ou externe du plateau mobile 4, voire la luxation de ce plateau, les contraintes ligamentaires, notamment celles dues aux ligaments latéraux, doivent être équilibrées de chaque côté interne et externe du genou prothésé.

Pour implanter une prothèse du genou telle que la prothèse 1, le chirurgien utilise successivement et/ou simultanément plusieurs ancillaires différents. Par exemple, parmi eux, on peut citer un ancillaire de résection de l'extrémité supérieure du tibia T.

De même, il est connu, par exemple de WO-A-01/85038, d'utiliser une tige introduite à l'intérieur du canal médullaire du fémur, depuis son extrémité inférieure. La manipulation de cette tige permet de contrôler, avant la solidarisation de la coiffe fémorale, que la coupe distale est perpendiculaire à l'axe mécanique du fémur, repéré par une radio pré-opératoire qui n'est pas toujours précise, par exemple en raison d'un flexum du patient. Cependant, on comprend qu'une telle tige de contrôle ne peut être introduite et sollicitée que lorsque le genou est fléchi, c'est-à-dire lorsque l'extrémité inférieure du fémur est accessible suivant l'axe du fémur. Ainsi, une telle tige intra-médullaire ne permet pas de contrôler l'équilibre ligamentaire en extension. Quant à la comparaison des encombrements prothétiques en flexion et en extension, elle est appréciée approximativement par le chirurgien. De plus, la mise en place de la tige intra-médullaire conduit généralement à un saignement postopératoire et à un risque septique accru.

Pour quantifier la tension ligamentaire en extension, il est connu, également de WO-A-01/85038, d'utiliser un autre ancillaire, de mise sous tension de l'articulation du genou, appelé couramment un ancillaire distracteur. Cet ancillaire se présente généralement sous la forme de deux branches articulées l'une par rapport à l'autre et dont les extrémités distales sont respectivement pourvues de moyens d'appui tibial et de moyens d'appui condylien. En interposant entre les parties proximales des branches un mécanisme d'écartement relatif, il est possible, après avoir disposé à l'intérieur de l'espace fémoro-tibial les extrémités distales des branches, de mettre sous tension l'articulation du genou, notamment en extension. Grâce, par exemple, à des moyens de mesure dynamométrique différentielle entre les deux condyles fémoraux, le chirurgien peut vérifier qu'aucun déséquilibre ligamentaire significatif n'est présent. Ce type d'ancillaire distracteur est cependant encombrant et compliqué à manipuler, ce qui décourage généralement le chirurgien qui doit manipuler successivement, et si nécessaire plusieurs fois de suite, la tige intra-médullaire évoquée plus haut et l'ancillaire distracteur, ce qui allonge les temps opératoires.

Le but de la présente invention est de proposer un ensemble d'ancillaires pour implanter une prothèse du genou, qui soit facile et rapide d'utilisation, en particulier de par le faible nombre d'ancillaires à manipuler, et qui améliore la précision de pose de la prothèse, notamment en ce qui concerne les encombrements prothétiques et l'équilibre ligamentaire latéral à la fois en flexion à 90° et en extension.

A cet effet, l'invention a pour objet un ensemble d'ancillaires pour implanter une prothèse de genou, comportant, entre autres, un premier ancillaire de distraction du genou, qui comprend, d'une part, deux branches mobiles l'une par rapport à l'autre et respectivement munies, à leur extrémité distale, de moyens d'appui tibial et de moyens d'appui fémoral et, d'autre part, des moyens d'écartement relatif des extrémités distales des branches, caractérisé en ce que les moyens d'appui tibial définissent une surface d'appui tibial sensiblement plane et en ce que l'ancillaire de distraction est muni en outre d'un dispositif permettant de repérer, d'au moins un côté interne ou externe du fémur, l'implantation d'au moins une broche extra-médullaire ou analogue suivant une direction appartenant à un plan sensiblement parallèle à la surface plane d'appui tibial et situé à une distance réglable de cette surface d'appui tibial.

Avec le seul ancillaire de distraction, le chirurgien peut à la fois vérifier l'équilibre ligamentaire interne/externe du genou en flexion à 90° et en extension et imposer automatiquement des encombrements prothétiques égaux en flexion et en extension. En effet, d'une part, les extrémités distales des branches de cet ancillaire peuvent être introduites dans l'espace fémoro-tibial d'implantation afin de distraire le genou, tant en flexion qu'en extension, et, d'autre part, le dispositif de repérage permet par exemple l'implantation réglée, de chaque côté interne et externe du fémur, d'au moins une paire, en pratique de plusieurs paires et de préférence trois, de broches extra-médullaires destinées à former des repères spatiaux pour les découpes ultérieures de l'extrémité inférieure du fémur, notamment au niveau de ses parties distale et postérieure. En conservant le même réglage d'écartement entre la face de résection tibiale et, d'une part, une première implantation de broches extra-médullaires alors que le genou est fléchi à 90° et, d'autre part, une seconde implantation de broches extra-médullaires alors que le genou est en extension, le chirurgien dispose de repères de coupes fémorales en vue de garantir des encombrements prothétiques égaux en flexion et en extension. Le plan contenant les broches implantées alors que le genou est en flexion est sensiblement perpendiculaire à l'axe de tension ligamentaire en flexion et sensiblement parallèle à l'axe de la corticale antérieure du fémur tandis que le plan contenant les broches implantées alors que le genou est en extension est sensiblement perpendiculaire à l'axe de tension ligamentaire en extension.

Suivant d'autres caractéristiques de cet ensemble d'ancillaires, prises isolément ou selon toutes les combinaisons techniquement possibles :

- le dispositif de repérage est apte à repérer, à la fois du côté interne et du côté externe du fémur, l'implantation d'au moins une paire de broches extra-médullaires ou analogues suivant des directions respectives appartenant à un même plan sensiblement parallèle à la surface plane d'appui tibial et situé à une distance réglable de cette surface d'appui ;
- le dispositif de repérage est apte à repérer, d'un même côté du fémur, l'implantation de deux broches extra-médullaires ou analogues suivant des directions respectives appartenant au même plan sensiblement parallèle à la surface plane d'appui tibial ;
- le dispositif de repérage comporte des moyens de visée extra-fémorale des directions d'implantation des broches ou analogues, ces moyens de visée délimitant par exemple des trous de guidage extra-médullaire d'un moyen pour creuser des cavités de réception des broches ou analogues ;
- l'ancillaire de distraction comporte une tige solidaire de la branche munie des moyens d'appui tibial et qui s'étend en longueur suivant une direction sensiblement perpendiculaire au plan contenant la surface plane d'appui tibial tandis que le dispositif de repérage comporte des moyens de liaison mobile entre cette tige et les moyens de visée ;
- les moyens de liaison mobile comportent des premiers moyens de déplacement des moyens de visée par rapport à la tige en translation le long de cette tige et le dispositif de repérage comporte des moyens de réglage et de blocage de la position en translation des moyens de visée ;
- l'ancillaire de distraction est pourvu d'un moyen de mesure de la position en translation des moyens de visée par rapport à la tige, par exemple sous forme de graduations portées par cette tige ;
- les moyens de réglage comprennent un palpeur de la corticale antérieure du fémur ;
- les moyens de liaison mobile comportent des deuxième moyens de déplacement des moyens de visée par rapport à la tige en rotation autour de l'axe longitudinal de cette tige ;
- les moyens de liaison mobile comportent des troisième moyens de déplacement des moyens de visée par rapport à la tige suivant deux directions sensiblement perpendiculaires à la direction longitudinale de la tige et sensiblement perpendiculaires entre elles ;
- il comporte une pièce de visée extra-médullaire de la tête fémorale, adaptée pour s'étendre sensiblement parallèlement à la direction longitudinale de la tige ;
- les moyens d'appui fémoral définissent une surface allongée convexe d'appui fémoral entre les condyles fémoraux, dont la dimension transversale est de préférence inférieure à 9 mm environ ;
- il comporte un second ancillaire de coupe fémorale, équipé de moyens de positionnement par rapport au fémur, adaptés pour coopérer avec des parties, en saillie des côtés interne et externe du fémur, de broches ou analogues implantées dans le fémur suivant les directions définies par le dispositif de repérage de l'ancillaire de distraction ;

- l'ancillaire de coupe délimite au moins une fente plane de coupe postérieure et une fente plane de coupe distale et les moyens de positionnement comportent à la fois une première paire de surfaces d'appui sur certaines desdites broches ou analogues, sensiblement parallèles à la fente de coupe postérieure et une seconde paire de surfaces d'appui sur d'autres desdites broches ou analogues, sensiblement parallèles à la fente de coupe distale, la distance entre ladite première paire de surfaces d'appui et le plan de la fente de coupe postérieure étant sensiblement égale à la distance entre ladite seconde paire de surfaces d'appui et le plan de la fente de coupe distale.

L'invention a également pour objet une méthode de pose d'une prothèse de genou, qui comporte les étapes successives durant lesquelles :

- on résèque l'extrémité supérieure du tibia ;
- au moyen d'un ancillaire de distraction, on distrait l'articulation du genou en flexion à 90° et on vérifie l'équilibre ligamentaire entre les côtés interne et externe du genou, notamment par comparaison visuelle des espaces rétro-condyliens interne et externe ;
- au moyen du même ancillaire de distraction, on distrait l'articulation du genou en extension et on vérifie l'équilibre ligamentaire entre les côtés interne et externe du genou, notamment en utilisant une pièce de visée extra-médullaire de la tête fémorale, rapportée sur cet ancillaire ;
- grâce à un dispositif de repérage porté par l'ancillaire de distraction et alors que le genou en flexion est distrait par cet ancillaire, on repère, d'au moins un côté interne ou externe du fémur, l'implantation d'au moins une broche extra-médullaire ou analogue, suivant une direction appartenant à un plan à la fois sensiblement parallèle au plan de résection tibiale, sensiblement perpendiculaire à l'axe de tension ligamentaire et situé à une distance donnée de cette surface, réglée à partir de la position de la corticale antérieure du fémur ;
- grâce au même dispositif de repérage porté par l'ancillaire et alors que le genou en extension est distrait par cet ancillaire, on repère, d'au moins un côté interne ou externe du fémur, l'implantation d'au moins une broche extra-médullaire ou analogue, suivant une direction appartenant à un plan à la fois sensiblement parallèle au plan de résection tibiale, sensiblement perpendiculaire à l'axe de tension ligamentaire et situé à la distance réglée de cette surface ;
- on positionne un ancillaire de coupe sur le fémur par coopération de cet ancillaire de coupe avec des parties, en saillie des côtés interne et externe du fémur, de broches extra-médullaires implantées dans le fémur selon les repérages précédents ; et
- on réalise des coupes du fémur, notamment distale et postérieure.

Avantageusement, les étapes de vérification de l'équilibre ligamentaire en flexion et en extension et les étapes de repérage d'implantation en flexion et en extension sont effectuées avec la rotule du patient globalement en place.

L'invention sera mieux comprise à la lecture de la description qui va suivre, donnée uniquement à titre d'exemple et faite en se référant aux dessins sur lesquels :

- la figure 1 est une vue éclatée en perspective d'un ancillaire de distraction appartenant à un ensemble d'ancillaires suivant l'invention ;
- la figure 2 est une vue en perspective illustrant la distraction d'un genou en flexion à 90°, par l'ancillaire de la figure 1 ;
- la figure 3 est une vue analogue à la figure 2, illustrant la distraction du même genou en extension, par l'ancillaire de la figure 1 ;
- la figure 4 est une vue en élévation de l'ancillaire de la figure 1, en cours d'utilisation sur le genou en flexion, afin de repérer l'implantation de broches fémorales extra-médullaires ;
- la figure 5 est une vue analogue à la figure 4, illustrant l'utilisation de l'ancillaire de la figure 1 sur le genou en extension, afin de repérer l'implantation d'autres broches fémorales extra-médullaires ;
- la figure 6 est une vue en élévation d'un ancillaire de coupe fémorale appartenant à l'ensemble d'ancillaires selon l'invention, positionné sur le fémur muni de broches implantées suivant les repérages des figures 4 et 5 ;
- la figure 7 est une vue en coupe de la figure 6, suivant un plan sagittal sensiblement médian d'un des condyles du fémur ;
- la figure 8A est une vue éclatée en perspective d'une prothèse de genou à plateau mobile ; et
- les figures 8B et 8C sont des vues en élévation, prises en interne, du genou pourvu de la prothèse de la figure 8A, respectivement en flexion et en extension.

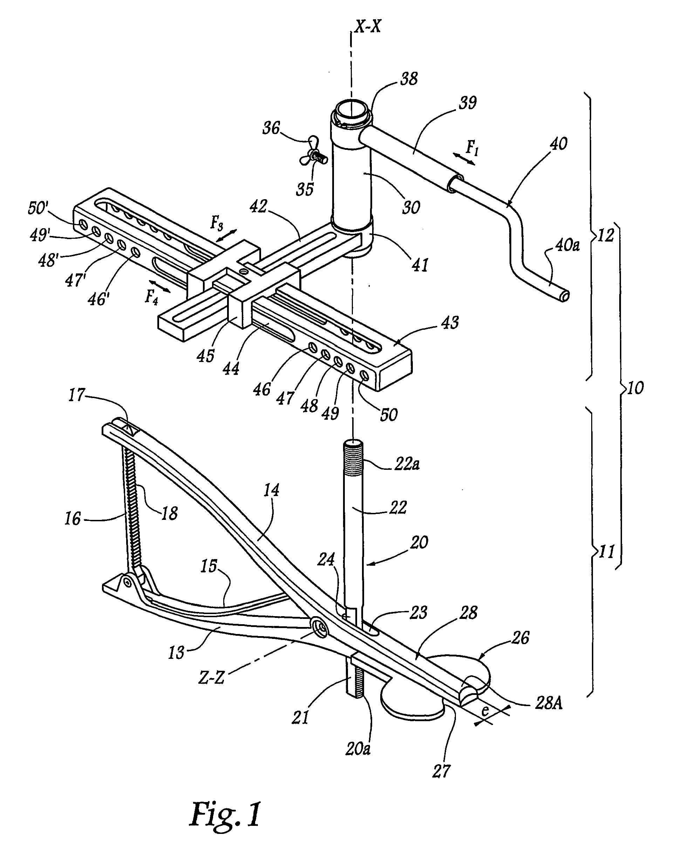

Sur la figure 1 est représenté un ancillaire 10 de distraction du genou, destiné à être utilisé pour implanter la prothèse 1 de la figure 8A. Cet ancillaire 10 comporte essentiellement un dispositif principal de distraction 11 et un dispositif de repérage 12 apte à être rapporté de manière amovible sur le dispositif principal 11, comme expliqué plus loin en détail.

Le dispositif principal 11 comporte deux branches rigides allongées 13 et 14, de forme courbe suivant leur longueur. Ces branches sont articulées en rotation l'une par rapport à l'autre autour d'un axe Z-Z sensiblement perpendiculaire à la direction longitudinale des branches. Entre les parties proximales des branches, c'est-à-dire leur partie destinées à être tournées vers le chirurgien lors de l'utilisation du dispositif 11, est interposée une lame de ressort 15 dimensionnée pour solliciter en permanence les branches de manière à ce que leur partie proximale s'écartent l'une de l'autre, autrement dit pour que leur partie distale se rapprochent l'une de l'autre. Pour permettre le réglage et le maintien d'une configuration rapprochée des parties proximales des branches, et donc une configuration écartée des parties distales de ces branches, le dispositif 11 est équipé d'une lame rigide 16. L'extrémité inférieure de la lame 16 est fixée par un axe à la branche inférieure 13 tandis que son extrémité supérieure est reçue dans une échancrure longitudinale correspondante 17 de la branche supérieure 14. La face distale de cette lame 16 est conformée en une crémaillère dont les dents 18 sont adaptées pour mettre en prise le fond distal de l'échancrure 17.

Dans sa partie distale, la branche inférieure 13 est solidaire d'une tige rigide 20 globalement cylindrique à base circulaire d'axe X-X et qui s'étend sensiblement perpendiculairement à la fois à la direction longitudinale de cette branche et à l'axe Z-Z. Cette tige 20 est par exemple soudée à la branche 13 ou bien est directement venue de matière avec elle.

La tige 20 comporte une partie inférieure 21 qui s'étend en saillie vers le bas de la branche inférieure 13, ainsi qu'une partie supérieure 22 qui s'étend en saillie vers le haut de cette même branche et qui présente une longueur supérieure à la partie inférieure 21. La partie supérieure 22 traverse de part en part la partie distale de la branche supérieure 14, à travers un trou oblong 23 de dimension la plus grande dirigée suivant la longueur de la branche 14 et au niveau duquel la partie de tige 22 présente deux méplats correspondants 24 diamétralement opposés et appartenant à des plans sensiblement perpendiculaires à l'axe Z-Z. La distance entre ces méplats 24 est sensiblement égale à la dimension la plus petite du trou 23 de sorte qu'aucun débattement transversal significatif n'est permis entre la tige et la branche 14.

A son extrémité distale, la branche inférieure 13 est solidaire d'une palette métallique 26, par exemple venue de matière avec le reste de la branche. Cette palette se présente sous la forme d'une plaque plane, rigide et de faible épaisseur par rapport au reste de la branche. Elle définit ainsi une face inférieure plane 26A qui appartient à un plan sensiblement perpendiculaire à l'axe X-X et qui est destinée à venir s'appuyer contre la surface réséquée $T_A$ du tibia T. A cet effet, le contour périphérique de la palette 26 est conformée de manière à s'adapter au mieux à la géométrie courante d'une résection tibiale, notamment en ménageant une échancrure distale 27 prévue pour recevoir les ligaments postérieurs de l'articulation du genou lors de la mise en place de la palette dans l'espace fémoro-tibial.

A son extrémité distale, la branche supérieure 14 se présente sous la forme d'un doigt rigide 28 venu de matière avec le reste de la branche, dans le prolongement longitudinal de cette dernière. La surface inférieure de ce doigt est sensiblement plane, ce qui permet un contact plan-plan avec la face supérieure de la palette 26 lorsque les parties proximales des branches 13 et 14 sont écartées au maximum comme à la figure 1.

La surface supérieure 28A du doigt 28 est de forme convexe et est dimensionnée pour pouvoir être logée dans l'échancrure inter-condylienne anatomique du fémur, comme expliqué plus loin. Cette surface est par exemple semi-cylindrique et sa largeur e est inférieure à environ 9 mm.

Comme indiqué plus haut, le dispositif de repérage 12 est adapté pour être rapporté de manière amovible sur le dispositif 11, plus précisément autour de sa tige 20. Ce dispositif 10 comporte un manchon tubulaire 30 de diamètre intérieur sensiblement égal au diamètre extérieur de la partie supérieure 22 de la tige 20. Le manchon 30 est ainsi rapporté autour de la partie de tige 22 de manière mobile à la fois en rotation autour de l'axe X-X et en translation le long de cet axe. Pour immobiliser le manchon par rapport à la tige, une vis de blocage 35 est prévue pour se coincer contre la face extérieure de la tige 20 en étant vissée dans un alésage radial au manchon 30. La tête de cette vis 35 est avantageusement munie d'oreilles 36.

A son extrémité supérieure, le manchon 30 est entouré par une bague extérieure 37 libre en rotation et fixe en translation par rapport au manchon. En saillie radiale de cette bague s'étend un tube 39 venu de matière avec la bague et qui reçoit une tige coudée 40. La partie de la tige 40 introduite dans le tube 39 est librement déplaçable en translation à l'intérieur de ce tube, comme indiqué par la double flèche $F_1$. Cette partie de tige délimite un méplat prévu pour s'étendre en regard d'un orifice radial non représenté délimité dans le tube 39. A l'intérieur de cet orifice, une vis de blocage peut être introduite pour bloquer la tige 40 par rapport au tube de façon à ce que l'axe de sa partie coudée 40a s'étende perpendiculairement à l'axe X-X.

A son extrémité inférieure, le manchon 30 est entouré par une autre bague extérieure 41, également libre en rotation et fixe en translation par rapport au manchon. En saillie radiale de cette bague, s'étend en longueur un rail 42 venu de matière avec la bague. Sur le rail 42, une barre de visée 43 est montée à coulissement libre suivant la longueur du rail, comme représenté par la double flèche $F_3$ à la figure 1. Plus précisément, cette barre délimite, suivant sa longueur, une rainure traversante 44 de forme oblongue, à l'intérieur de laquelle est reçu le rail 42. La longueur de cette rainure 44 est dimensionnée pour permettre le déplacement relatif en translation de la barre 43 par rapport au rail 42 suivant la direction longitudinale de la barre, comme représenté par la double flèche $F_4$, c'est-à-dire suivant une direction sensiblement orthoradiale par rapport à l'axe X-X. Pour éviter que la barre 43 ne se désengage totalement du rail 42, et pour rigidifier la liaison entre le rail et la barre, un étrier 45 de rattrapage des jeux est prévu. Une vis non rerpésentée de blocage est rapportée sur l'étrier pour immobiliser de manière temporaire le rail 42 et la barre 43 l'un par rapport à l'autre.

Dans chaque partie d'extrémité de la barre 43 sont creusés des trous traversants 46 à 50 et 46' à 50' répartis suivant la longueur de la barre. Les trous 46 à 50 sont symétriques aux trous 46' à 50' par rapport au plan médian de la barre 43 parallèle à l'axe X-X, les trous 46 et 46' étant les deux trous situés les plus près de la rainure 44, de chaque côté de cette dernière. Les axes respectifs de ces trous 46 à 50 et 46' à 50' s'étendent transversalement à la direction longitudinale de la barre 43 et appartiennent tous à un même plan sensiblement perpendiculaire à l'axe X-X lorsque le manchon 30 est rapporté sur la tige 20.

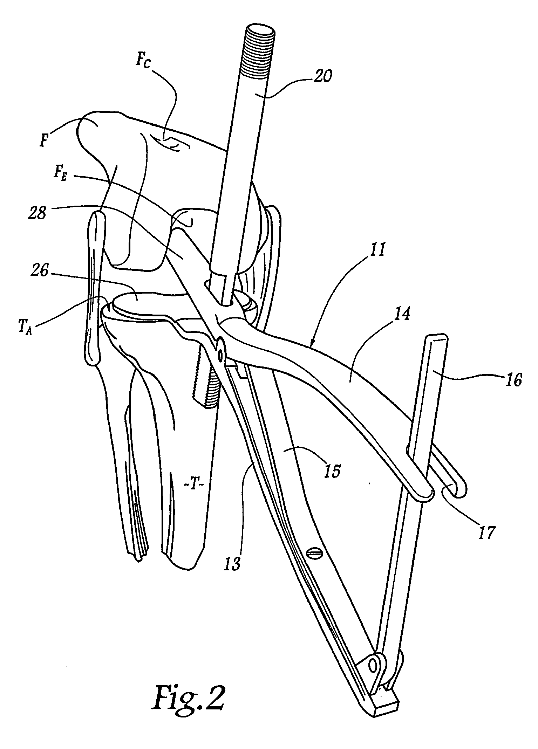

L'utilisation de l'ancillaire de distraction 10 représentée à la figure 1 va maintenant être décrite en regard des figures 2 à 5, en vue d'implanter la prothèse 1 de la figure 8A.

Après avoir incisé la zone du genou d'un patient à opérer et en considérant par exemple que la voie d'abord privilégiée est interne, le chirurgien résèque l'extrémité supérieure du tibia T, par exemple au moyen d'un ancillaire de coupe tibiale conventionnel. A la fin de cette étape préalable, le tibia T présente, à son extrémité supérieure, la surface réséquée $T_A$ sensiblement plane, en principe sensiblement perpendiculaire à l'axe mécanique du tibia qui passe par la zone centrale de cette surface et la cheville correspondante du patient. L'extrémité inférieure du fémur F est pour le moment intacte.

Dans un premier temps, le chirurgien loge à l'intérieur de l'espace fémoro-tibial les extrémités distales des branches 13 et 14 du dispositif 11. Plus précisément, comme représenté à la figue 2, la surface inférieure 26A de la palette 26 est mise en appui contre la surface réséquée $T_A$ du tibia T, tandis que le doigt 28 est glissé à l'aplomb de l'échancrure inter-condylienne $F_E$ du fémur F. Alors que le genou est en flexion à 90°, le chirurgien rapproche les parties proximales des branches 13 et 14 de l'ancillaire 1 de manière à ce que le doigt 28 se loge entre les condyles interne et externe du fémur et écarte progressivement le fémur F du tibia T. Lorsque la tension d'écartement entre le tibia et le fémur correspond à une tension anatomiquement appropriée, le chirurgien bloque la branche supérieure 14 par rapport à la branche inférieure 13 au moyen d'une des dents 18 de la lame 16. Le dispositif 11 de l'ancillaire 10 est alors dans la configuration de la figure 2.

Le chirurgien a alors accès aux espaces rétro-condyliens de l'articulation et, si nécessaire, résèque des ostéophytes tibiaux et/ou fémoraux postérieurs et/ou décolle les ligaments latéraux des flancs des condyles et des coques condyliennes. Dans le cas où le chirurgien constate visuellement que l'un des espaces rétro-condyliens est plus grand que l'autre, ce qui indique qu'un différentiel de tensions ligamentaires subsiste de part et d'autre de l'articulation du genou, il procède à des corrections chirurgicales appropriées, notamment par l'élimination supplémentaire d'ostéophytes et/ou par des décollements ligamentaires plus étendus.

On remarquera qu'il est nécessaire de luxer la rotule du patient pour pouvoir observer les espaces rétro-condyliens. Cependant, une fois les corrections chirurgicales effectuées, la rotule peut être réduite, la présence du doigt effilé 28 à l'extrémité de la branche supérieure 14 ne gênant pas la remise en place de la rotule. L'équilibre ligamentaire est ainsi contrôlé sans perturbation significative de l'environnement ligamentaire du genou.

Par ailleurs, s'il existait, avant l'opération, une subluxation, généralement externe, de la rotule du patient, le chirurgien peut être amené à dégager de la matière osseuse condylienne postérieure de façon plus importante en interne qu'en externe en conservant une égalité des espaces rétro-condyliens externe et interne. La coiffe fémorale 3 de la prothèse 1 ultérieurement implantée sera alors légèrement décalée en rotation vers l'extérieur de manière à être centré vis-à-vis de la rotule du patient.

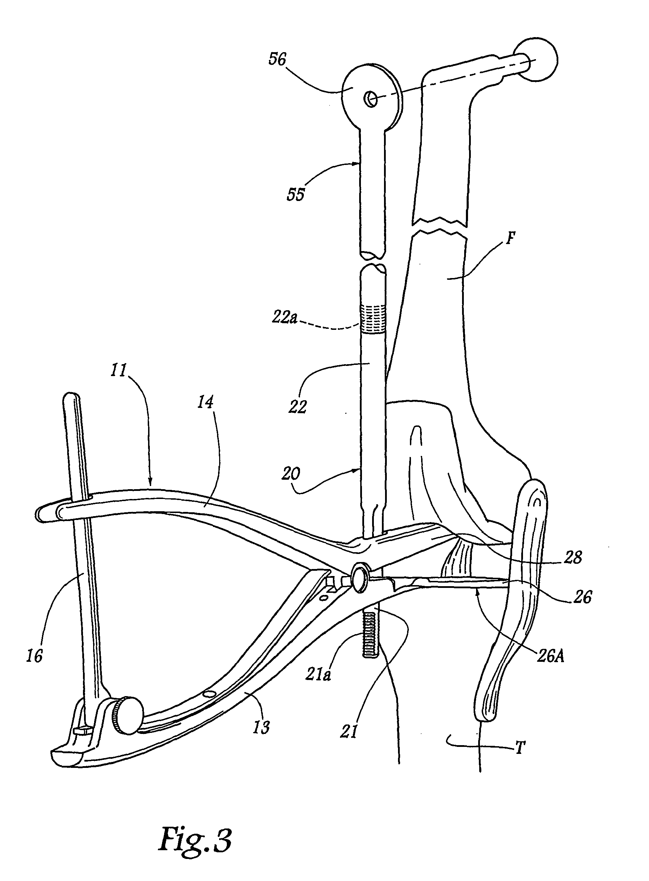

Dans un deuxième temps, une fois l'équilibre ligamentaire en flexion ainsi contrôlé, le chirurgien détend l'ancillaire de distraction 10 et amène l'articulation du genou en extension, comme à la figure 3. Comme précédemment, il distrait alors cette articulation en écartant les extrémités distales des branches 13 et 14 jusqu'à ce que cette articulation soit soumise à une contrainte de distraction sensiblement opposée à la tension ligamentaire anatomique. Le chirurgien rapporte ensuite à l'extrémité supérieure de la tige 20, une tige 55 de visée fémorale. Cette tige 55 est par exemple vissée autour d'un filetage correspondant 22a de la partie de tige 22. A l'extrémité libre de la tige 55 est prévu un moyen de visée 56 de la tête anatomique du fémur F. Si ce viseur 56 se projette en regard du centre de la tête fémorale, repérée par exemple par une radio per-opératoire, le chirurgien conclut que l'axe mécanique du fémur, c'est-à-dire l'axe passant par ce centre et la zone inter-condylienne $F_E$ du fémur, est sensiblement confondu avec l'axe de traction ligamentaire en extension. Dans le cas contraire, le chirurgien résèque d'éventuels ostéophytes postérieurs qui gênent les coques condyliennes du genou.

De manière optionnelle et non représentée, le chirurgien peut vérifier que l'axe mécanique du fémur et l'axe mécanique du tibia sont bien sensiblement confondus en rapportant sur le dispositif 11 une tige supplémentaire de visée tibiale. Cette tige supplémentaire est, à une extrémité, par exemple vissée autour d'un filetage correspondant 21a de la partie de tige 21 tandis que son extrémité opposée s'étend jusqu'à la cheville du patient.

Comme pour le premier temps opératoire, une fois l'articulation distraite, la rotule est réduite pendant la vérification de l'équilibre ligamentaire.

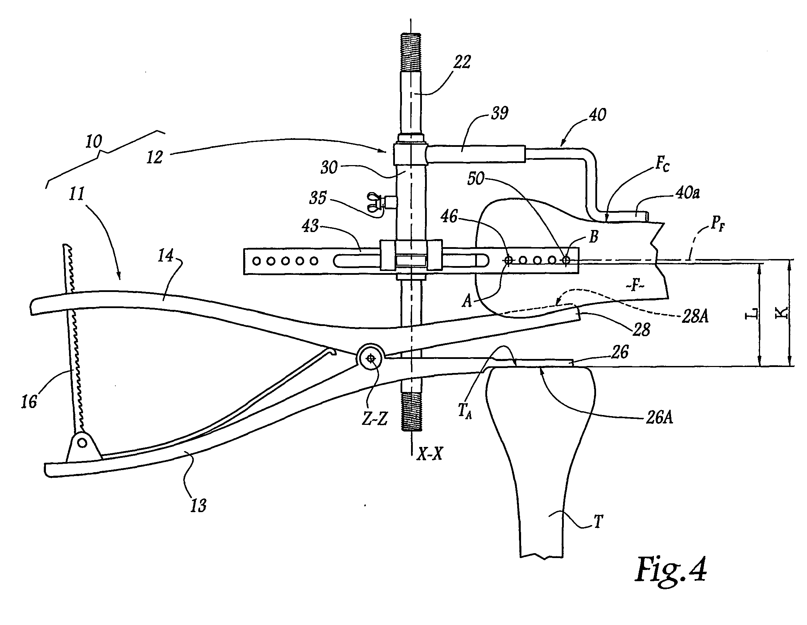

Dans un troisième temps, une fois que l'équilibre ligamentaire en extension est ainsi obtenu, l'ancillaire de distraction 10 est détendu et le genou est alors amené de nouveau en configuration fléchie à 90°. De la même façon que durant l'étape de contrôle de l'équilibre ligamentaire en flexion, les branches 13 et 14 de l'ancillaire 10 sont sollicitées de manière à distraire l'articulation du genou jusqu'à ce que l'intensité de distraction soit sensiblement opposée à la tension ligamentaire anatomique. Le dispositif de repérage 12, qu'il n'était pas jusqu'alors nécessaire de rapporter sur le dispositif 11, est rapporté autour de la tige 20. Le tube 39 de la bague supérieure 38 est déplacé pour s'étendre sensiblement dans le prolongement longitudinal du fémur tandis que le manchon 30 est déplacé en translation vers le bas le long de la tige 20 jusqu'à ce que la partie coudée 40a de la tige 40, formant ainsi un palpeur, vienne au contact de la corticale antérieure $F_C$ du fémur F, comme représenté à la figure 4, où elle est bloquée. On remarquera que la forme coudée de la tige 40 permet de surplomber le massif épyphisaire du fémur, sans être gêné par celui-ci. Le manchon 30 est alors immobilisé en translation le long de la tige 20 au moyen de la vis de blocage 35. La barre 43 est ensuite entraînée, d'abord en rotation par rapport au manchon 30 de manière à ce que l'une de ses parties d'extrémité, par exemple celle munie des trous 46 à 50, soit disposée en regard du flanc interne du fémur, puis en translation par rapport au rail 42 de sorte que le trou 46 soit situé, suivant la direction antéro-postérieure, à quelques millimètres de la face d'extrémité distale du fémur F. La distance séparant alors le bord inférieur du trou 46 de la surface 26A d'appui tibial est noté L sur la figure 4.

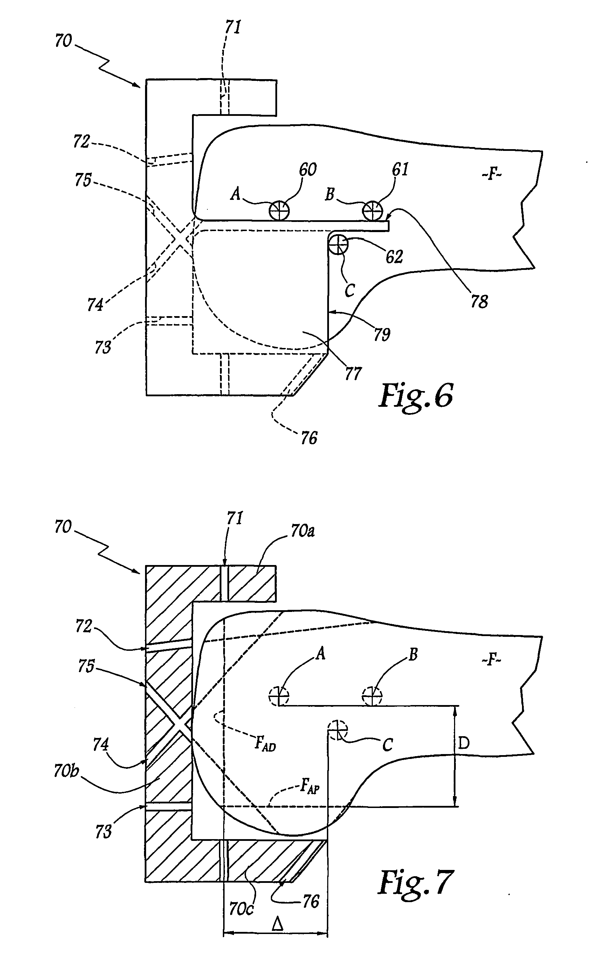

Le chirurgien introduit alors le foret d'une perceuse à l'intérieur du trou 46 de manière à forer une cavité extra-médullaire dans l'os, d'axe noté A et de quelques dizaines de millimètres de profondeur. Il creuse également une autre cavité, d'axe B, en introduisant le foret dans un des autres trous 47 à 50 de la barre de visée 43, par exemple le trou 50 à la figure 4, de façon à creuser le massif épiphysaire fémoral et non sa diaphyse plus compacte. Dans chacune des cavités extra-médullaires ainsi creusées, il implante une broche cylindrique (référencée respectivement 60 et 61 à la figure 6), en laissant saillante une partie de la broche.

Tout en conservant le réglage en translation du manchon 30 sur la tige 20, la bague 41 est entraînée en rotation autour de l'axe X-X de manière à amener l'autre partie d'extrémité de la barre de visée 43 contre le flanc externe du fémur F. Deux autres cavités sont alors creusées dans l'épiphyse fémorale en guidant le foret de perçage respectivement dans les trous 46' et 50'. Deux autres broches sont ensuite implantées dans ces cavités extra-médullaires, avec des parties respectives en saillie du côté externe du fémur.

Lors de chaque guidage du foret de perçage par l'un des trous de la barre de visée 43, la position en rotation de la bague 41 par rapport à la tige 20 et la position en translation de la barre 43 le long du rail 42 peuvent être ajustées de manière à ce que la barre 43 soit plaquée au plus près du flanc latéral du fémur F. Il s'ensuit que les axes longitudinaux A et B des cavités creusées ne sont pas nécessairement parallèles les uns aux autres. En revanche, ces axes appartiennent tous à un même plan sensiblement parallèle à la surface 26A d'appui tibial, indiqué par sa trace $P_F$ à la figure 4 et situé, par rapport à la surface 26A, à une distance K liée au réglage de la position en translation du manchon 30 le long de la tige 20 et égale à la distance L augmentée du rayon des trous 46 à 50 et 46' à 50'. Ce plan $P_F$ est ainsi à la fois parallèle à la partie de surface de la corticale antérieure $F_C$ du fémur F sur laquelle la partie coudée 40a de la tige 40 a été placée en contact pour régler la position en translation du manchon 30 et sensiblement perpendiculaire à l'axe de traction ligamentaire en flexion en raison de l'équilibre ligamentaire recherché au premier temps opératoire et du débattement en rotation de l'échancrure intercondylienne $F_E$ sur la surface 28a du doigt 28.

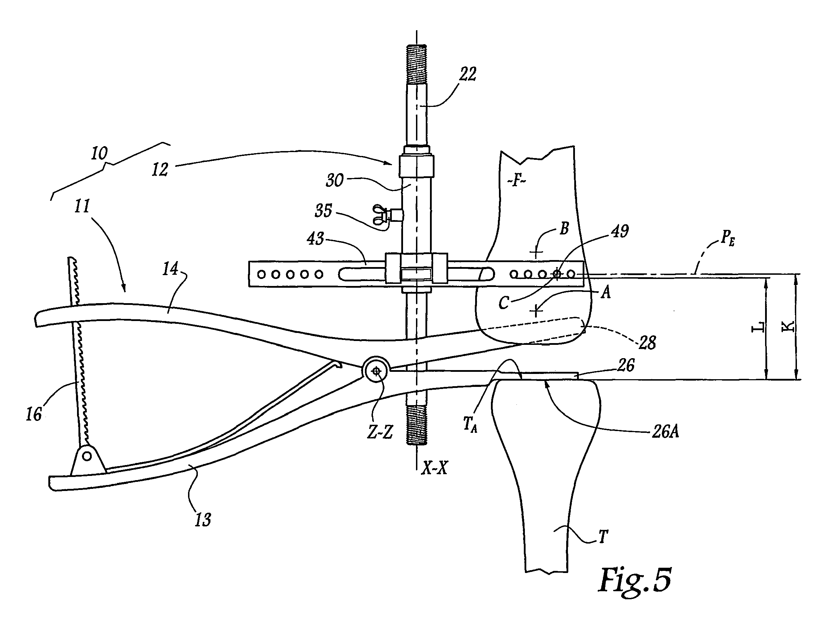

Dans un quatrième temps, toujours sans modifier le positionnement en translation du manchon 30 par rapport à la tige 20, la tige-palpeur 40 est pivotée, le dispositif principal de distraction 11 est détendu, l'articulation du genou est passée en extension puis le dispositif 11 est retendu de manière à distraire l'articulation, comme durant le second temps opératoire. Comme représenté à la figue 5, l'un des trous de chaque série de trous 46 à 50 et 46' à 50' de la barre 43 est alors utilisé de la même façon que précédemment, c'est-à-dire pour guider l'application du foret de perçage précité de manière à réaliser, de chaque côté interne et externe du fémur F, une cavité extra-médullaire à l'intérieur de laquelle est implantée une broche (référencée 62 à la figure 6 pour le flanc interne) analogue aux broches précitées, en partie saillante des flancs interne et externe du fémur. A la figure 5, c'est par exemple les trous 49 et 49' qui sont utilisés et l'axe de la cavité creusée du côté interne est noté C. Comme précédemment, les axes respectifs des deux cavités extra-médullaires interne et externe s'étendent alors dans un plan sensiblement parallèle à la surface 26A d'appui tibial, situé à la distance K de cette surface et indiqué par sa trace $P_E$ à la figure 5. Ce plan $P_E$ est ainsi sensiblement perpendiculaire à, à la fois, l'axe mécanique du fémur et l'axe de tension ligamentaire en extension en raison de l'équilibre ligamentaire en extension recherché au deuxième temps opératoire et du débattement en rotation de l'échancrure intercondylienne $F_E$ sur la surface 28a du doigt 28.

On remarquera que, durant les troisième et quatrième temps opératoires, la rotule est laissée en place ou est tout au plus légèrement décalée vers l'extérieur pour permettre le passage des parties distales des branches 13 et 14.

Dans un sixième temps, après avoir retiré l'ancillaire 10 et luxé la rotule, un bloc de coupe 70 est positionné sur le fémur F, comme représenté aux figures 6 et 7. Ce bloc de coupe fémoral 70 est adapté pour guider des lames de coupe du fémur de manière à réaliser des plans de résection fémorale adaptés aux plans de la face 3A du capot fémoral 3 à implanter.

Plus précisément, le bloc 70 présente, dans sa partie courante, une section globalement en forme de C ouvert de façon à loger l'extrémité du fémur F, genou fléchi à 90°, comme représenté sur la figure 7. Ce bloc délimite :

- dans sa branche supérieure 70a, une fente 71 de coupe distale,
- dans sa base 70b, une fente 72 de coupe antérieure et une double-fente 73 de coupe postérieure, chaque fente élémentaire de cette double-fente étant prévue pour un des condyles fémoraux, ainsi que des fentes 74 et 75 de coupes chanfreinées destinées à réaliser des coupes inclinées reliant respectivement les coupes antérieure et distale et les coupes postérieure et distale, et
- dans sa branche inférieure 70c, une double-fente 76 de coupe extra-postérieure, destinée à réaliser des coupes condyliennes débouchant sur les coupes postérieures et inclinées vers l'avant par rapport à ces dernières, par exemple d'environ 45°.

On remarquera que les fentes distale 71 et postérieure 73 sont sensiblement perpendiculaires l'une par rapport à l'autre, de sorte que les coupes réalisées au moyen de ces fentes sont celles prévues pour s'étendre sensiblement parallèlement à la surface réséquée $T_A$ du tibia T lorsque le genou est respectivement en extension (comme à la figure 8C) et en flexion (comme à la figure 8B).

Les coupes condyliennes destinées à être réalisées au moyen de la double-fente 76 ne sont pas prévues pour correspondre à une partie de la face multi-plans 3A du capot 3 mais sont adaptées pour permettre la suppression d'éventuels surplus osseux responsables de conflits entre la partie postérieure de l'extrémité réséquée $F_A$ du fémur et le bord postérieur du plateau mobile 4 lorsque le genou prothésé est en flexion extrême.

Pour permettre d'obtenir des plans de coupes fémorales convenablement positionnés en vue de l'implantation de la prothèse 1, le bloc 70 est muni de flancs latéraux 77 destinés à être disposés de chaque côté interne et externe du fémur et symétriques par rapport à un plan médian du bloc, perpendiculaire à la fente postérieure 73. Chaque flanc 77 présente, d'une part, une première surface plane d'appui 78 parallèle à la fente postérieure 73 et qui, lorsque le bloc est positionné sur le fémur, s'étend le long du massif épiphysaire fémoral et, d'autre part, une seconde surface plane d'appui 79 sensiblement perpendiculaire à la surface 78, autrement dit sensiblement parallèle à la fente distale 71. Ces surfaces 78 et 79 s'étendent à une même distance à de respectivement le plan de la fente postérieure 73 et le plan de la fente distale 71. Cette distance Δ est prédéterminée en fonction de la taille de la coiffe fémorale 3 à implanter. Dans l'exemple considéré, la distance Δ est égale à la distance AP (figure 8B) entre le point d'extrémité supérieure du plan antérieure $3A_A$ de la face multi-plans 3A de la coiffe 3 et le plan postérieur $3A_P$ de cette face multi-plans 3A, à laquelle est soustraite une constante J pré-déterminée, fonction de dimensions fixes du dispsoitif 12. La constante J est égale à la distance, suivant l'axe X-X, entre la face inférieure du palpeur 40a et le plan passant par les axes des trous 46 à 50 et 46' à 50', augmentée du rayon de ces trous. Ainsi, à chaque taille d'implant fémoral, c'est-à-dire à chaque distance AP, est associé un bloc de coupe analogue au bloc 70. En utilisation, comme représenté sur les figures 6 et 7, le bloc de coupe 70 est positionné sur le fémur F de façon à ce que la surface 78 soit plaquée contre les broches 60 et 61 tandis que la surface 79 est plaquée contre les broches 62.

Lorsque ce positionnement est réalisé, le bloc de coupe 70 est, si nécessaire, immobilisé par rapport au fémur, par exemple au moyen de fiches ou de poignées d'immobilisation non-représentées qui traversent chaque flanc 77 de l'ancillaire et qui s'ancrent dans l'os. En option non représentée, chaque flanc 77 est équipé d'une barre parallèle à la surface 78 correspondante et mobile suivant une direction transversale à cette surface. Cette barre est ainsi déplaçable par rapport à la surface 78 de manière à pouvoir enserrer les broches 60 et 61, un moyen de blocage de la barre étant prévu pour l'immobiliser. Le bloc de coupe est ainsi davantage stabilisé sur le fémur, pour limiter autant que possible l'utilisation d'autres moyens d'immobilisation invasifs.

Des lames de coupe ou de scie sont alors introduites dans les différentes fentes de l'ancillaire. En particulier, l'introduction d'une telle lame dans la fente postérieure 73 permet la résection postérieure du fémur F suivant le plan de coupe $F_{AP}$ disposé à une distance Δ des broches 60 et 61 et l'introduction dans la fente 71 permet la résection distale du fémur suivant le plan de coupe $F_{AD}$ disposé à une distance Δ de la broche 62. En conséquence, d'une part, la distance séparant le plan de coupe postérieure $F_{AP}$ de la surface tibiale $T_A$ est égale à Le lorsque le genou est fléchi à 90° comme à la figure $8_B$ et, d'autre part, la distance séparant le plan de coupe distale $F_{AD}$ de cette surface tibiale $T_A$ est également égale à L-Δ lorsque le genou est en extension comme à la figure 8C. Autrement dit, les encombrements prothétiques en flexion $EP_1$ et en extension $EP_2$ obtenus en utilisant le bloc de coupe 70 positionné sur le fémur F par les broches 60 à 62 sont sensiblement égaux entre eux et vérifient la relation

$$EP_1 \approx EP_2 = L\text{-}\Delta = L - AP + J$$

Le plateau tibial 2 de la prothèse 1 est ensuite solidarisé au tibia T, le plateau mobile 4 est mis en place et la coiffe fémorale 3 est enfin solidarisée au fémur F.

L'utilisation des deux ancillaires 10 et 70 selon l'invention permet ainsi d'obtenir rapidement, facilement et avec précision des compartiments prothétiques en flexion et en extension de hauteurs sensiblement égales. Un seul aide est par exemple nécessaire au côté du chirurgien.

Les deux ancillaires 10 et 70 n'occupent qu'une place restreinte et sont utilisés sans difficultés par le chirurgien, sans augmenter de manière significative la durée de l'intervention chirurgicale, voire en la diminuant.

De plus, l'établissement d'un environnement ligamentaire exempt de sur-tension ou de déséquilibre en tension diminue le niveau de contraintes entre les composants métalliques 2 et 3 et le plateau en polyéthylène 4, tout au moins évite la formation de zones de sur-contraintes. L'usure du polyéthylène est donc diminuée, en tout cas ralentie. L'amorce de délaminations et l'apparition d'une décooptation interne ou externe sont également évitées.

Suivant un aménagement non représenté particulièrement avantageux de l'invention, la tige 20 présente, dans sa partie supérieure 22, une série de graduations adaptées pour quantifier le positionnement en translation du manchon 30 le long de cette tige lors de son immobilisation par la vis de blocage 35. A partir de cette mesure et en connaissant la taille du composant fémoral de la prothèse à implanter, le chirurgien est à même de déterminer, par exemple au moyen d'un abaque, l'épaisseur du plateau mobile à implanter. En effet, le repérage de la position du manchon le long de la tige 20 donne une information représentative de la distance L (ou K) définie plus haut tandis que le choix de la taille des composants tibial et fémoral de la prothèse à implanter permet de connaître à l'avance la valeur $\Delta$ associée à cette prothèse. De la sorte, il est possible de déterminer l'encombrement prothétique résultant de la différence entre la distance L et la valeur $\Delta$ et, par soustraction des épaisseurs de la coiffe fémorale 3 et de l'embase tibiale 2, d'obtenir la valeur de l'épaisseur du plateau 4 la plus appropriée, c'est-à-dire celle qui permet d'obtenir une épaisseur prothétique sensiblement égale à l'encombrement prothétique disponible en flexion et en extension. En cours d'intervention, le chirurgien peut donc décider du plateau 4 le plus approprié au patient opéré.

Dans l'hypothèse où le chirurgien ne dispose pas d'une gamme quasi-continue d'épaisseurs de plateau, mais plutôt d'une série de plateaux d'épaisseur graduellement croissante par un palier non négligeable, il se peut, par exemple, qu'il détermine, par la méthode décrite ci-dessus, que l'épaisseur la plus appropriée pour le plateau tibial à implanter est d'environ 9 mm alors qu'il ne dispose que de plateaux d'épaisseur de 8 et 10 mm. Dans ce cas, il est avantageusement prévu, selon l'invention, de disposer de plusieurs types de broches à implanter dans le massif épihysaire fémoral. Plus précisément, en plus des broches cylindriques 60 à 62 de diamètre extérieur sensiblement constant sur toute leur longueur, on dispose de broches cylindriques extérieurement étagées, c'est-à-dire dont la partie destinée à s'ancrer dans l'os est de diamètre sensiblement égal au diamètre des broches 60 à 62 tandis que la partie opposée est de diamètre extérieur légèrement plus petit et/ou légèrement plus grand que le diamètre extérieur des broches 60 à 62, d'environ 0,5, 1 ou 1,5 mm par exemple. De la sorte, en implantant de telles broches étagées de chaque côté interne et externe du fémur lors du cinquième temps opératoire, le chirurgien décale d'environ 1 mm la position du bloc de coupe 70 par rapport à la position qu'il aurait occupé si les broches 60 à 62 avaient été utilisées comme précédemment de sorte que l'un des plateaux d'épaisseur 8 ou 10 mm précité sera finalement implanté tout en s'adaptant au mieux à l'encombrement prothétique disponible.

En variante, plutôt que d'implanter des paires de broches comme décrit ci-dessus, le chirurgien peut implanter, d'un seul côté interne ou externe du fémur F, des broches suffisamment longues pour, en fin d'implantation, s'étendre en saillie de chaque côté du fémur. Dans ce cas, l'endommagement de l'épiphyse fémorale est plus important, mais, sous réserve que les cavités creusées pour recevoir ces broches transfixiantes soient bien rectilignes, une seule étape de repérage et d'implantation d'un des deux côtés interne ou externe du fémur, est suffisante, ce qui réduit encore davantage la durée de l'intervention.

De plus, que les broches implantées soient transfixiantes ou par paires, il peut être prévu de les entourer d'une gaine en matière plastique à usage unique pour empêcher de les ré-utiliser.

Par ailleurs, bien que les ancillaires 10 et 70 ont été décrits ci-dessus dans le but d'implanter la prothèse 1 à plateau mobile, ces ancillaires sont bien entendu utilisables pour implanter une prothèse de genou dépourvue d'un tel plateau mobile.

Divers aménagements et variantes aux ancillaires décrits ci-dessus sont en outre envisageables :

- le dispositif de distraction 11 peut être équipé d'un système de débrayage de la lame d'écartement 16 afin d'éviter de léser les ligaments du genou en cas d'une trop forte mise sous tension ;
- de même, des moyens de mesure dynamométrique peuvent être prévus entre les branches 13 et 14 pour quantifier la mise sous tension du genou ;
- la lame à dents 16 peut être remplacée par une tige filetée solidaire de la branche 13, parallèle à l'axe X-X et autour de laquelle une molette, liée à la branche 14, est déplaçable, à la façon d'un système vis écrou ; dans ce cas, la pièce 55 de visée extra-médullaire de la tête fémorale peut être rapportée à l'extrémité libre de cette tige filetée ;
- au lieu de l'unique barre de visée 43 mobile en rotation autour de l'axe X-X par rapport au manchon 30, le dispositif de repérage 12 peut comporter deux barres de visée, fixes en rotation par rapport au manchon 30 et disposées de part et d'autre de l'axe X-X ; dans ce cas, la bague 41 est par exemple venue de matière avec le manchon 30 et deux rails 42, reliés chacun de manière coulissante à l'une de ces deux barres de visée, sont prévus symétriquement de chaque côté du manchon 30 ;

- une bague cylindrique à base circulaire peut être montée à libre rotation autour de l'extrémité libre du doigt 28 de manière à faciliter le basculement du fémur au niveau de son échancrure inter-condylienne $F_E$ par rapport à la branche 14 ;

- l'actionnement du dispositif de distraction 11 peut être inversé, c'est-à-dire qu'on peut prévoir que, au repos, les extrémités distales des branches 13 et 14 sont écartées l'une de l'autre par un ressort de compression interposé entre les parties proximales de ces branches tandis que, en comprimant ce ressort, on rapproche ces parties distales l'une de l'autre de manière à permettre leur introduction dans l'espace fémoro-tibial ; dans ce cas, le ressort de compression précité est dimensionné pour que, lors du relâchement des parties proximales des branches de l'ancillaire, la mise sous tension de l'articulation soit réalisée avec une valeur anatomiquement appropriée ;

- les ancillaires 10 et 70 peuvent être adaptés à la navigation assistée par ordinateur ; et/ou

- le bloc de coupe 70 n'est pas nécessairement réalisé d'un seul tenant ; en particulier, il est possible d'utiliser un sous-bloc de coupe existant, muni d'au moins une fente de coupe postérieure et de monter rigidement ce sous-bloc sur un cadre aménagé selon l'invention, dans lequel est notamment prévue une fente de coupe distale et dont les flancs sont analogues aux flancs 77 décrits plus haut.

**Revendications**

1. Ensemble d'ancillaires (10, 70) pour implanter une prothèse de genou (1), comportant, entre autres, un premier ancillaire (10) de distraction du genou, qui comprend, d'une part, deux branches (13, 14) mobiles l'une par rapport à l'autre et respectivement munies, à leur extrémité distale, de moyens d'appui tibial (26) et de moyens d'appui fémoral (28) et, d'autre part, des moyens (16) d'écartement relatif des extrémités distales des branches, **caractérisé en ce que** les moyens d'appui tibial (26) définissent une surface d'appui tibial (26A) sensiblement plane et **en ce que** l'ancillaire de distraction (10) est muni en outre d'un dispositif (12) permettant de repérer, d'au moins un côté interne ou externe du fémur (F), l'implantation d'au moins une broche extra-médullaire (60 ; 61 ; 62) ou analogue suivant une direction (A ; B ; C) appartenant à un plan ($P_F$ ; $P_E$) sensiblement parallèle à la surface plane d'appui tibial (26A) et situé à une distance réglable (K) de cette surface d'appui tibial.

2. Ensemble d'ancillaires suivant la revendication 1, **caractérisé en ce que** le dispositif de repérage (12) est apte à repérer, à la fois du côté interne et du côté externe du fémur (F), l'implantation d'au moins une paire de broches extra-médullaires (60 ; 61 ; 62) ou analogues suivant des directions respectives (A ; B ; C) appartenant à un même plan ($P_F$ ; $P_E$) sensiblement parallèle à la surface plane d'appui tibial (26A) et situé à une distance réglable (K) de cette surface d'appui.

3. Ensemble d'ancillaires suivant l'une des revendications 1 ou 2, **caractérisé en ce que** le dispositif de repérage (12) est apte à repérer, d'un même côté du fémur, l'implantation de deux broches extra-médullaires ou analogues (60, 61) suivant des directions respectives (A, B) appartenant au même plan ($P_F$) sensiblement parallèle à la surface plane d'appui tibial (26A).

4. Ensemble d'ancillaires suivant l'une quelconque des revendications précédentes, **caractérisé en ce que** le dispositif de repérage (12) comporte des moyens (43) de visée extra-fémorale des directions (A ; B ; C) d'implantation des broches (60 ; 61 ; 62) ou analogues, ces moyens de visée (43) délimitant par exemple des trous (46 à 50, 46' à 50') de guidage extra-médullaire d'un moyen pour creuser des cavités de réception des broches ou analogues.

5. Ensemble d'ancillaires suivant la revendication 4, **caractérisé en ce que** l'ancillaire de distraction (10) comporte une tige (20) solidaire de la branche (13) munie des moyens d'appui tibial (26) et qui s'étend en longueur suivant une direction (X-X) sensiblement perpendiculaire au plan contenant la surface plane d'appui tibial (26A) et **en ce que** le dispositif de repérage (12) comporte des moyens (30, 41, 42, 44, 45) de liaison mobile entre cette tige et les moyens de visée (43).

6. Ensemble d'ancillaires suivant la revendication 5, **caractérisé en ce que** lesdits moyens de liaison mobile comportent des premiers moyens (30) de déplacement des moyens de visée (43) par rapport à la tige (20) en translation le long de cette tige et **en ce que** le dispositif de repérage (12) comporte des moyens de réglage (40) et de blocage (36) de la position en translation des moyens de visée.

7. Ensemble d'ancillaires suivant la revendication 6, **caractérisé en ce que** l'ancillaire de distraction (10) est pourvu d'un moyen de mesure de la position en translation des moyens de visée (43) par rapport à la tige (20), par exemple sous forme de graduations portées par cette tige.

8. Ensemble d'ancillaires suivant l'une des revendications 6 ou 7, **caractérisé en ce que** lesdits moyens de réglage (40) comprennent un palpeur (40a) de la corticale antérieure ($F_C$) du fémur (F).

9. Ensemble d'ancillaires suivant l'une quelconque des revendications 5 à 8, **caractérisé en ce que** les moyens de liaison mobile comportent des deuxième moyens (41) de déplacement des moyens de visée (43) par rapport à la tige (20) en rotation autour de l'axe longitudinal (X-X) de cette tige.

10. Ensemble d'ancillaires suivant l'une quelconque des revendications 5 à 9, **caractérisé en ce que** les moyens de liaison mobile comportent des troisième moyens (42, 44) de déplacement des moyens de visée (43) par rapport à la tige (20) suivant deux directions ($F_3$, $F_4$) sensiblement perpendiculaires à la direction longitudinale (X-X) de la tige et sensiblement perpendiculaires entre elles.

11. Ensemble d'ancillaires suivant l'une quelconque des revendications 5 à 10, **caractérisé en ce qu'**il comporte une pièce (55) de visée extra-médullaire de la tête fémorale, adaptée pour s'étendre sensiblement parallèlement à la direction longitudinale (X-X) de la tige (20).

12. Ensemble d'ancillaires suivant l'une quelconque des revendications précédentes, **caractérisé en ce que** les moyens d'appui fémoral (28) définissent une surface allongée convexe (28A) d'appui fémoral entre les condyles fémoraux, dont la dimension transversale (e) est de préférence inférieure à 9 mm environ.

13. Ensemble d'ancillaires suivant l'une quelconque des revendications précédentes, **caractérisé en ce qu'**il comporte un second ancillaire (70) de coupe fémorale, équipé de moyens (78, 79) de positionnement par rapport au fémur (F), adaptés pour coopérer avec des parties, en saillie des côtés interne et externe du fémur (F), de broches ou analogues (60 à 62) implantées dans le fémur suivant les directions (A, B, C) définies par le dispositif de repérage (12) de l'ancillaire de distraction (10).

14. Ensemble d'ancillaires suivant la revendication 13, **caractérisé en ce que** l'ancillaire de coupe (70) délimite au moins une fente plane (73) de coupe postérieure et une fente plane (71) de coupe distale et **en ce que** les moyens de positionnement comportent à la fois une première paire de surfaces (78) d'appui sur certaines (60, 61) desdites broches ou analogues (60 à 62), sensiblement parallèles à la fente de coupe postérieure (73) et une seconde paire de surfaces (79) d'appui sur d'autres (62) desdites broches ou analogues, sensiblement parallèles à la fente de coupe distale (71), la distance (Δ) entre ladite première paire de surfaces d'appui (78) et le plan de la fente de coupe postérieure (73) étant sensiblement égale à la distance (Δ) entre ladite seconde paire de surfaces d'appui (79) et le plan de la fente de coupe distale (71).

**Claims**

1. A set of instruments (10, 70) for implanting a knee prosthesis (1), the set including amongst others, a first instrument (10) for distracting the knee, which instrument comprises firstly two branches (13, 14) movable relative to each other and respectively provided at their distal ends with means (26) for pressing against the tibia and with means (28) for pressing against the femur, and secondly means (16) for moving the distal ends of the branches mutually apart, the set being **characterized in that** the means (26) for pressing against the tibia define a tibial bearing surface (26A) that is substantially plane, and **in that** the distraction instrument (10) is further provided with a device (12) serving to locate, on at least one of the medial and lateral sides of the femur (F), the position for implanting at least one extramedullary pin (60; 61; 62) or the like in a direction (A; B; C) lying in a plane ($P_F$; $F_E$) that is substantially parallel to the plane tibial bearing surface (26A) and that is situated at an adjustable distance (K) from said tibial bearing surface.

2. A set of instruments according to claim 1, **characterized in that** the locator device (12) is suitable, both on the medial side and on the lateral side of the femur (F), for locating the positions for implanting at least one pair of extramedullary pins (60; 61; 62) or the like along respective directions (A; B; C) lying in a common plane ($P_F$; $P_E$) that is substantially parallel to the plane tibial surface (26A) and that is situated at an adjustable distance (K) from said bearing surface.

**3.** A set of instruments according to claim 1 or claim 2, **characterized in that** the locator device (12) is suitable, on a given side of the femur, for locating the positions for implanting two extramedullary pins or the like (60, 61) in respective directions (A, B) lying in a common plane ($P_F$) substantially parallel to the plane tibial bearing surface (26A).

**4.** A set of instruments according to any preceding claim, **characterized in that** the locator device (12) includes extra-femoral jig means (43) for defining the directions (A; B; C) along which the pins (60; 61; 62) or the like are implanted, these jig means (43) defining, for example, extramedullary guide holes (46 to 50, 46' to 50') for means for forming cavities for receiving the pins or the like.

**5.** A set of instruments according to claim 4, **characterized in that** the distraction instrument (10) includes a rod (20) secured to the branch (13) provided with the means (26) for pressing against the tibia, and extending lengthwise along a direction (X-X) that is substantially perpendicular to the plane containing the plane tibial bearing surface (26A), and **in that** the locator device (12) includes moving connection means (30, 41, 42, 44, 45) between said rod and the jig means (43).

**6.** A set of instruments according to claim 5, **characterized in that** said moving connection means comprise first means (30) for moving the jig means (43) relative to the rod (20) in translation along said rod, and **in that** the locator device (12) includes adjustment and locking means (40, 36) for adjusting and locking the position in translation of the jig means.

**7.** A set of instruments according to claim 6, **characterized in that** the distraction instrument (10) is provided with means for measuring the position in translation of the jig means (43) relative to the rod (20), e.g. in the form of graduations carried by said rod.

**8.** A set of instruments according to claim 6 or claim 7, **characterized in that** said adjustment means (40) comprise a feeler (40a) for feeling the anterior cortex ($F_c$) of the femur (F).

**9.** A set of instruments according to any one of claims 5 to 8, **characterized in that** the moving connection means comprise second means (41) for moving the jig means (43) relative to the rod (20) in pivoting about the longitudinal axis (X-X) of said rod.

**10.** A set of instruments according to any one of claims 5 to 9, **characterized in that** the moving connection means include third means (42, 44) for moving the jig means (43) relative to the rod (20) in two directions ($F_3$, $F_4$) that are substantially perpendicular to the longitudinal direction (X-X) of the rod and substantially perpendicular to each other.

**11.** A set of instruments according to any one of claims 5 to 10, **characterized in that** it includes an extramedullary sight part (55) for sighting the head of the femur and adapted to extend substantially parallel to the longitudinal direction (X-X) of the rod (20).

**12.** A set of instruments according to any preceding claim, **characterized in that** the means (28) for pressing against the femur define a convex elongate surface (28A) for pressing against the femur between the condyles thereof, and having a transverse dimension ($\underline{e}$) that is preferably less than about 9 mm.

**13.** A set of instruments according to any preceding claim, **characterized in that** it includes a second instrument (70) for cutting the femur, fitted with means (78, 79) for positioning the instrument relative to the femur (F), that are adapted to co-operate with projecting portions of pins or the like (60 to 62) implanted in the femur along directions (A, B, C) defined by the locator device (12) of the distraction instrument (10), said projecting portions projecting from the medial and lateral sides of the femur (F).

**14.** A set of instruments according to claim 13, **characterized in that** the cutter instrument (70) defines at least a plane posterior cutting slot (73) and a plane distal cutting slot (71), and **in that** the positioning means comprise both a first pair of bearing surfaces (78) for bearing against some (60, 61) of said pins or the like (60 to 62), substantially parallel to the posterior cutting slot (73), and a second pair of bearing surfaces (79) for bearing against others (62) of said pins or the like, substantially parallel to the distal cutting slot (71), the distance ($\Delta$) between said first pair of bearing surfaces (78) and the plane of the posterior cutting slot (73) being substantially equal to the distance ($\Delta$) between said second pair of bearing surfaces (79) and the plane of the distal cutting slot (71).

**Patentansprüche**

1. Einheit von Hilfsmitteln (10, 70) zum Implantierten einer Knieprothese (1), die unter anderem ein erstes Hilfsmittel (10) zur Distraktion des Knies aufweist, das einerseits zwei Schenkel (13, 14) umfasst, die in Bezug aufeinander beweglich und an ihrem distalen Ende jeweils mit Tibia-Stützeinrichtungen (26) und Femur-Stützeinrichtungen (28) und andererseits mit Einrichtungen (16) zum relativen Spreizen der distalen Enden der Schenkel ausgestattet sind, **dadurch gekennzeichnet, dass** die Tibia-Stützeinrichtungen (26) eine im Wesentlichen plane Tibia-Stützfläche (26A) bilden, und dass das Distraktionshilfsmittel (10) außerdem mit einer Vorrichtung (12) ausgestattet ist, die es ermöglicht, zumindest auf einer Innen- oder Außenseite des Femurs (F), die Implantation mindestens eines extramedullären Stifts (60; 61; 62) oder dergleichen entlang einer Richtung (A; B; C) zu markieren, die zu einer Ebene ($P_F$; $P_E$) gehört, die zur planen Tibia-Stützfläche (26A) im Wesentlichen parallel ist und sich in einem einstellbaren Abstand (K) von dieser Tibia-Stützfläche befindet.

2. Einheit von Hilfsmitteln nach Anspruch 1, **dadurch gekennzeichnet, dass** die Markierungsvorrichtung (12) gleichzeitig auf der Innenseite und auf der Außenseite des Femurs (F) die Implantation mindestens eines Paars von extramedullären Stiften (60; 61; 62) oder dergleichen entlang jeweiliger Richtungen (A; B; C) markieren kann, die zu derselben Ebene ($P_F$; $P_E$) gehören, die zur planen Tibia-Stützfläche (26A) im Wesentlichen parallel ist und sich in einem einstellbaren Abstand (K) von dieser Tibia-Stützfläche befindet.

3. Einheit von Hilfsmitteln nach einem der Ansprüche 1 oder 2, **dadurch gekennzeichnet, dass** die Markieruugsvorrichtung (12) auf derselben Seite des Femurs die Implantation zweier extramedullärer Stifte (60, 61) oder dergleichen entlang jeweiliger Richtungen (A; B; C) markieren kann, die zu derselben Ebene ($P_F$) gehören, die zur planen Tibia-Stützfläche (26A) im Wesentlichen parallel ist.

4. Einheit von Hilfsmitteln nach einem der vorhergehenden Ansprüche, **dadurch gekennzeichnet, dass** die Markierungsvorrichtung (12) Einrichtungen (43) zur extrafemuralen Anvisierung der Richtungen (A; B; C) der Implantation der Stifte (60; 61; 62) oder dergleichen umfasst, wobei diese Visiereinrichtungen (43) beispielsweise Öffnungen (46 bis 50, 46' bis 50') zur extramedullären Führung einer Einrichtung zum Ausheben der Aufnahmehohlräume für die Stifte oder dergleichen eingrenzen.

5. Einheit von Hilfsmitteln nach Anspruch 4, **dadurch gekennzeichnet, dass** das Distraktionshilfsmittel (10) einen fest mit dem Schenkel (13) verbundenen Stab (20) umfasst, der mit den Tibia-Stützeinrichtungen (26) ausgestattet ist, und der sich der Länge nach entlang einer Richtung (X-X) erstreckt, die im Wesentlichen senkrecht zu der Ebene ist, welche die plane Tibia-Stützfläche (26A) enthält, und dass die Markierungsvorrichtung (12) Einrichtungen (30, 41, 42, 44, 45) zur beweglichen Verbindung zwischen diesem Stab und den Visiereinrichtungen (43) umfasst.

6. Einheit von Hilfsmitteln nach Anspruch 5, **dadurch gekennzeichnet, dass** die Einrichtungen zur beweglichen Verbindung erste Einrichtungen (30) zur Translationsverschiebung der Visiereinrichtungen (43) in Bezug auf den Stab (20) entlang dieses Stabs umfassen, und dass die Markierungsvorrichtung (12) Einrichtungen zur Einstellung (40) und Arretierung (36) der Translationsstellung der Visiereinrichtungen umfasst.

7. Einheit von Hilfsmitteln nach Anspruch 6, **dadurch gekennzeichnet, dass** das Distraktionshilfsmittel (10) mit einer Messeinrichtung für die Translationsstellung der Visiereinrichtungen (43) in Bezug auf den Stab (20), beispielsweise in Form von an diesem Stab angebrachten Gradeinteilungen versehen ist.

8. Einheit von Hilfsmitteln nach einem der Ansprüche 6 oder 7, **dadurch gekennzeichnet, dass** die Einstelleinrichtungen (40) einen Taster (40a) für die vordere Kortikalis ($F_c$) des Femurs (F) umfassen.

9. Einheit von Hilfsmitteln nach einem der Ansprüche 5 bis 8, **dadurch gekennzeichnet, dass** die Einrichtungen zur beweglichen Verbindung zweite Einrichtungen (41) zur Drehverschiebung der Visiereinrichtungen (43) in Bezug auf den Stab (20) um eine Längsachse (X-X) dieses Stabs umfassen.

10. Einheit von Hilfsmitteln nach einem der Ansprüche 5 bis 9, **dadurch gekennzeichnet, dass** die Einrichtungen zur beweglichen Verbindung dritte Einrichtungen (42, 44) zur Verschiebung der Visiereinrichtungen (43) in Bezug auf den Stab (20) entlang zweier Richtungen ($F_3$, $F_4$) umfassen, die im Wesentlichen senkrecht zur Längsrichtung (X-X) des Stabs und zueinander im Wesentlichen senkrecht sind.

11. Einheit von Hilfsmitteln nach einem der Ansprüche 5 bis 10, **dadurch gekennzeichnet, dass** sie ein Teil (55) zur extramedullären Anvisierung des Femurkopfes umfasst, das dazu ausgelegt ist, sich im Wesentlichen parallel zur Längsrichtung (X-X) des Stabs (20) zu erstrecken.

**12.** Einheit von Hilfsmitteln nach einem der vorhergehenden Ansprüche, **dadurch gekennzeichnet, dass** die Femur-Stützeinrichtungen (28) eine längliche konvexe Fläche (28A) zur Femur-Abstützung zwischen den Femurkondylen bilden, deren Querabmessung (e̲) vorzugsweise kleiner als ca. 9 mm ist.

**13.** Einheit von Hilfsmitteln nach einem der vorhergehenden Ansprüche, **dadurch gekennzeichnet, dass** sie ein zweites Hilfsmittel (70) für einen Femurschnitt umfasst, das mit Einrichtungen (78, 79) zur Positionierung in Bezug auf den Femur (F) ausgestattet ist, die dazu ausgelegt sind, mit von der Innen- und Außenseite des Femurs (F) vorstehenden Teilen von Stiften (60 bis 62) oder dergleichen zusammenzuwirken, die in den Femur entlang der Richtungen (A, B, C) implantiert sind, die durch die Markierungsvorrichtung (12) des Distraktionshilfsmittels (10) vorgegeben werden.

**14.** Einheit von Hilfsmitteln nach Anspruch 13, **dadurch gekennzeichnet, dass** das Schnitthilfsmittel (70) mindestens einen planen Schlitz (73) zum hinteren Schnitt und einen planen Schlitz (71) zum distalen Schnitt eingrenzt, und dass die Positionierungseinrichtungen gleichzeitig ein erstes Paar von Flächen (78) zur Abstützung an bestimmten (60, 61) der Stifte (60 bis 62) oder dergleichen, die im Wesentlichen parallel zum hinteren Schnittschlitz (73) sind, und ein zweites Paar von Flächen (79) zur Abstützung an anderen (62) der Stifte und dergleichen umfassen, die im Wesentlichen parallel zum distalen Schnittschlitz (71) sind, wobei der Abstand (Δ) zwischen dem ersten Paar von Stützflächen (78) und der Ebene des vorderen Schnittschlitzes (73) im Wesentlichen gleich dem Abstand (Δ) zwischen dem zweiten Paar von Stützflächen (79) und der Ebene des distalen Schnittschlitzes (71) ist.

*Fig.1*

*Fig.2*

Fig.3

Fig.4

Fig.5

EP 1 732 448 B1

Fig.6

Fig.7

Fig.8A          Fig.8B          Fig.8C

EP 1 732 448 B1

**EP 1 732 448 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0185038 A